# FASCICULE DE BREVET EUROPEEN

(11) **EP 3 541 478 B1**
(45) Date de publication et mention de la délivrance du brevet: **06.01.2021**
(21) Numéro de dépôt: 17811991.3
(22) Date de dépôt: 16.11.2017
(51) Int. Cl.: A61K 36/58, A61K 8/9789, A61P 17/00, A61Q 19/02

(54) **UTILISATION COSMÉTIQUE, NUTRACEUTIQUE OU PHARMACEUTIQUE PRÉFÉRENTIELLEMENT DERMATOLOGIQUE D'UN EXTRAIT DE FEUILLES DE LA PLANTE LANSIUM DOMESTICUM POUR DIMINUER LA PIGMENTATION DE LA PEU ET/OU DES ANNEXES CUTANÉES**
KOSMETISCHE, NUTRAZEUTISCHE ODER PHARMAZEUTISCHE, VORZUGSWEISE DERMATOLOGISCHE VERWENDUNG EINES EXTRAKTES AUS BLÄTTERN DER LANSIUM-DOMESTICUM-PFLANZE ZUR REDUZIERUNG DER PIGMENTIERUNG DER HAUT UND/ODER DER HAUTANHANGSGEBILDE
COSMETIC, NUTRACEUTICAL OR PHARMACEUTICAL - PREFERABLY DERMATOLOGICAL - USE OF AN EXTRACT OF LEAVES FROM THE LANSIUM DOMESTICUM PLANT FOR REDUCING THE PIGMENTATION OF THE SKIN AND/OR SKIN APPENDAGES

(30) Priorité: 17.11.2016 FR 1661162
(43) Date de publication de la demande: 25.09.2019
(73) Titulaire: BASF Beauty Care Solutions France SAS, 69007 Lyon (FR)
(72) Inventeur: DEGRAVE, Véronique, 69160 Tassin la Demi Lune (FR); REYMERMIER, Corinne, 69390 Charly (FR)
(74) Mandataire: Cabinet Becker et Associés
(86) Numéro de dépôt international: PCT/FR2017/053129
(87) Numéro de publication internationale: WO 2018/091825

(56) Documents cités:
- ARUNG ENOS TANGKE ET AL: "Evaluation of medicinal plants from Central Kalimantan for antimelanogenesis.", JOURNAL OF NATURAL MEDICINES OCT 2009, vol. 63, no. 4, octobre 2009 (2009-10), pages 473-480, XP002770771, ISSN: 1861-0293 cité dans la demande
- TILAAR MARTHA ET AL: "Review of Lansium domesticum Correa and its use in cosmetics", BOLETIN LATINOAMERICANO Y DEL CARIBE DE PLANTAS MEDICINALES Y AROMATICAS, vol. 7, no. 4, juillet 2008 (2008-07), pages 183-189, XP002770772, cité dans la demande
- YAPP DONALD T T ET AL: "Lansium domesticum: Skin and leaf extracts of this fruit tree interrupt the lifecycle of Plasmodium falciparum, and are active towards a chloroquine-resistant strain of the parasite (T9) in vitro.", JOURNAL OF ETHNOPHARMACOLOGY, vol. 85, no. 1, mars 2003 (2003-03), pages 145-150, XP002770773, ISSN: 0378-8741

### SEQUENCE LISTING

<110> BASF Beauty Care Solutions FR SAS
<120> UTILISATION COSMETIQUE, NUTRACEUTIQUE OU PHARMACEUTIQUE, PREFERENTIELLEMENT DERMATOLOGIQUE D'UN EXTRAIT DE FEUILLES DE LA PLANTE LANSIUM DOMESTICUM POUR DIMINUER LA PIGMENTATION DE LA PEAU ET/OU DES ANNEXES CUTANEES
<130> B2357PC
<160> 2
<170> PatentIn version 3.5
<210> 1
   <211> 128
   <212> RNA
   <213> Homo sapiens
<400> 1
   uggaggccuu gcugguuugg aaaguucauu guucgacacc auggaucucc agguggguca        60
   aguuuagaga ugcaccaacc uggaggacuc caugcuguug agcuguucac aagcagcgga       120
   cacuucca                                                                128
<210> 2
   <211> 22
   <212> RNA
   <213> Homo sapiens
<400> 2
   caaccuggag gacuccaugc ug 22

## Description

La présente invention a pour objet l'utilisation cosmétique et/ou nutraceutique ou pharmaceutique, préférentiellement dermatologique d'un extrait de plante pour diminuer la pigmentation de la peau et/ou des annexes cutanées, et/ou diminuer les taches pigmentaires, et/ou pour traiter et/ou prévenir l'hyperpigmentation de la peau et/ou des annexes cutanées.

Le processus de pigmentation de la peau également dénommée « mélanogénèse » comprend de nombreuses étapes allant de la synthèse de la mélanine dans les mélanocytes à son transport dans les kératinocytes de l'épiderme par l'intermédiaire des mélanosomes. Plusieurs protéines interviennent dans ce processus. La tyrosinase (TYR) est la première enzyme responsable de la synthèse de mélanine et coopère avec deux autres protéines dénommées TYR-related protein 1 (TYRP1) et dopachrome tautomerase (TYRP2) pour transformer la mélanine en eumelanine et phoemelanine.

En outre, au cours du vieillissement de la peau, notamment sous l'effet des radiations, typiquement des ultraviolets, des taches brunes dite séniles ou pigmentaires, aussi appelées « aged spots », peuvent apparaitre caractérisant une accélération de la mélanogénèse, souvent jugées inesthétiques.

Il existe un besoin grandissant dans le domaine de la cosmétique d'élaboration d'ingrédients actifs capables de dépigmenter la peau pour des souhaits esthétiques, en particulier dans certaines régions du monde telle que l'Asie, au sein d'une population plus sujette aux taches pigmentaires. Ce besoin est d'autant plus important que les actifs cosmétiques dépigmentants connus sont majoritairement des molécules chimiques dont l'efficacité et l'innocuité sont parfois discutables. Par ailleurs, la plupart des actifs disponibles sur le marché réduisent la mélanogénèse par un effet d'inhibition directe de l'activité de la tyrosinase. Ainsi, le principal inconvénient de tels actifs est qu'ils ont un effet court terme car en leur absence, lorsqu'on arrête ou ralentie leur application, la protéine présente redevient active et reprend au même niveau d'activité qu'initialement. De tels actifs sont donc dénués d'effet long terme. L'élaboration d'actifs naturels végétaux et à effet durable dans le temps correspond à une demande forte en cosmétique.

Les inventeurs ont découvert de façon surprenante qu'un extrait de feuilles de la plante *Lansium domesticum* avait la capacité de diminuer la pigmentation de la peau et/ou des annexes cutanées, notamment en diminuant la mélanogénèse, tout particulièrement par une augmentation de l'expression génique du miRNA490, faisant de cet extrait un ingrédient alternatif d'intérêt en cosmétique répondant à la problématique à résoudre, en particulier par son effet durable dans le temps.

La plante *Lansium domesticum* est un arbre de la famille des Meliaceae originaire d'Asie du Sud Est, que l'on trouve en Thaïlande, Indonésie, Vietnam, mais aussi en Australie, Inde, Sri Lanka, ainsi que dans certaines îles d'Amérique centrale. La plante est connue en tant que plante médicinale. La pellicule du fruit, difficile à séparer du fruit, est riche en oléorésine, est utilisée contre les spasmes intestinaux. Les graines broyées sont utilisées pour traiter la fièvre et l'écorce pour la malaria. Le fruit est connu en cosmétique. Ainsi, un extrait de péricarpe a été utilisé dans une composition en tant qu'agent anti-acnéique (JP19870046320). La demande de brevet JP2001122731 divulgue par ailleurs un extrait de *L. domesticum* dans une composition cosmétique comme agent hydratant de la peau. Le fruit est aussi connu comme actif dépigmentant via son activité anti-tyrosinase et antioxydant (Tilaar MT., Review of Lansium domesticum Corrêa and its use in cosmetics, 2007, Boletin Latinoamericano y del Caribe de Plantas Médicinales y Aromaticas, 7, pages 183 à 189). Un extrait méthanolique d'écorce de *L. domesticum* a aussi montré sa capacité à inhiber la synthèse de mélanine par son effet anti-tyrosinase (L-DOPA et tyrosinase de champignon) et inhibition de synthèse de mélanine dans des mélanocytes B16 (Arung ET., Evaluation of medicinal plants from Central Kalimantan for antimelanogenesis, 2009, J. Nat. Med., 63, pages 473 à 780).

Toutefois à la connaissance de la demanderesse, aucune utilisation cosmétique des feuilles de *L. domesticum* en tant qu'agent dépigmentant n'a été décrite en particulier pour un effet durable.

La présente invention offre l'avantage de fournir un ingrédient actif dépigmentant efficace alternatif à ceux déjà connus. Ce nouvel ingrédient est par ailleurs extrait de plante non toxique, facilement formulable et qui peut être produit à l'échelle industrielle. En outre, ledit extrait de feuilles présente l'avantage important d'augmenter l'expression génique du miRNA490 ce qui, contrairement à des inhibiteurs d'activité de tyrosinase, permet d'obtenir un effet durable. Ceci permet de réduire également dans le temps la quantité et/ou la fréquence d'applications de l'actif. Enfin, dans la mesure où il s'agit d'un extrait de feuilles la présente invention offre l'avantage d'une matière première disponible tout au long de l'année et en grande quantité s'inscrivant dans une démarche de développement durable par l'utilisation de matière première renouvelable.

Un premier objet de l'invention concerne donc l'utilisation cosmétique et/ou nutraceutique d'un extrait de feuilles de *Lansium domesticum* pour diminuer la pigmentation de la peau et/ou des annexes cutanées, préférentiellement des cheveux et poils, et/ou pour diminuer les taches brunes de la peau.

On entend ici par « utilisation cosmétique et/ou nutraceutique » une utilisation non thérapeutique, non pharmaceutique, c'est-à-dire destinée à toute zone de peau et/ou annexe cutanée dite saine.

On entend par « zone de peau saine », une zone de peau sur laquelle est appliquée l'extrait selon l'invention dite « non pathologique » par un dermatologue, aussi qualifiée de « normale », c'est à dire qui ne présente pas d'infection, de cicatrice, de maladie ou d'affection cutanée telle que candidose, impétigo, psoriasis, eczéma, acné ou dermatite, ou de plaies ou de blessures et/ou autres dermatoses.

L'extrait est destiné à être appliqué sur tout ou partie du corps choisi parmi les mains, le cou, le décolleté, le ventre, les bras, les cuisses, les hanches, la taille et/ou le visage, et/ou les annexes cutanées, et préférentiellement les mains, le cou, le décolleté et/ou le visage, encore préférentiellement le visage.

On entend par « annexes cutanées » les cheveux, les poils, les ongles, préférentiellement les cheveux et/ou les poils, encore préférentiellement les poils.

On entend au sens de la présente invention par « diminuer la pigmentation de la peau et/ou des annexes cutanées» diminuer la quantité de mélanine d'au moins 50%, préférentiellement d'au moins 75%, encore préférentiellement d'au moins 85% en présence de l'extrait de *L. domesticum* selon l'invention, par rapport à la quantité de mélanine mesurée en l'absence de l'extrait. La quantité de mélanine peut être mesurée selon les techniques connues par l'homme du métier comme par exemple dans des mélanocytes B16, notamment en présence de l'extrait de *L. domesticum* tel que celui préparé selon l'exemple 1a), dans les conditions décrites selon l'exemple 5. Dans un mode de réalisation avantageux de l'invention, la mesure est effectuée par mesure de la densité optique à 475 nm. Selon un mode avantageux, la diminution de la pigmentation est durable dans le temps c'est-à-dire que le niveau de pigmentation après application de l'extrait selon l'invention se maintient au moins 2 jours, préférentiellement au moins 4 jours après l'arrêt de l'utilisation de l'extrait selon l'invention.

Dans un mode de réalisation particulièrement avantageux de l'invention, l'extrait selon l'invention augmente l'expression du miRNA490, également appelé hsa-mir-490, et défini dans la base de référence mribase.org sous la référence MI0003125 et présente une séquence pré-mature de SEQ ID No 1 : UGGAGGCCUUGCUGGUUUGGAAAGUUCAUUGUUCGACACCAUGGAUCU CCAGGUGGGUCAAGUUUAGAGAUGCACCAACCUGGAGGACUCCAUGCU GUUGAGCUGUUCACAAGCAGCGGACACUUCCA). Le miRNA mature est hsa-miR-490-3p de SEQ ID No 2 : CAACCUGGAGGACUCCAUGCUG. On entend par « augmenter l'expression du miRNA490 », augmenter l'expression génique du miRNA490. L'augmentation de l'expression du miRNA490 est d'au moins 1%, préférentiellement d'au moins 25%, encore préférentiellement d'au moins 40% en présence de l'extrait de *L. domesticum* selon l'invention, par rapport au niveau d'expression détectée en l'absence de l'extrait. Avantageusement, l'expression du miRNA490 est mesurée dans des mélanocytes, avantageusement des mélanocytes humains dits normaux, c'est-à-dire non pathologiques. Encore avantageusement, l'augmentation de l'expression du miRNA490 est mesurée par RT-PCR, encore avantageusement en présence ou non de l'extrait de *L. domesticum* préparé selon l'exemple 1a), dans les conditions décrites dans l'exemple 4.

Dans un mode de réalisation particulièrement avantageux de l'invention, l'extrait selon l'invention diminue l'expression génique et/ou protéique de la tyrosinase (TYR). On entend selon l'invention par « diminuer l'expression génique et/ou protéique de la tyrosinase (TYR) » provoquer une diminution d'au moins 15%, préférentiellement d'au moins 25%, encore préférentiellement d'au moins 37% de l'expression de TYR en présence de l'extrait de *L. domesticum* selon l'invention, par rapport au niveau d'expression mesurée en l'absence de l'extrait. Préférentiellement, il s'agit d'une diminution de l'expression protéique de TYR. Avantageusement encore, la mesure de l'expression protéique est effectuée dans des mélanocytes humains dits normaux, encore préférentiellement par Western bot, encore préférentiellement en présence de l'extrait de *L. domesticum* préparé selon l'exemple 1a), dans les conditions décrites dans l'exemple 3.

Différentes techniques de mesure *in vivo* peuvent en outre être utilisées pour mesurer la diminution de pigmentation de la peau et/ou des annexes cutanées et notamment la rémanence de cet effet. La mesure *in vivo* peut être effectuée par chromamétrie. Cette technique mesure l'absorbance à des longueurs d'onde distinctes (DO 420/520/620nm) et permet une mesure de 3 paramètres (L*, a* et b*) (L* représente la « clarté », a* représente la « rougeur » et b* représentant le « jaune »).

La diminution de la quantité de mélanine et/ou des tâches pigmentaires peut aussi être mesurée par un Siascope® (technique de siascopie), générant une analyse intracutanée spectrophotométrique haute résolution et permettant de mesurer notamment la concentration totale en mélanine dans l'épiderme.

L'utilisation de l'extrait de *L. domesticum* selon l'invention est donc pour diminuer la pigmentation de la peau et/ou des annexes cutanées, préférentiellement des poils, et/ou diminuer les taches brunes de la peau, avantageusement de manière durable.

On entend au sens de la présente invention par « diminuer les taches pigmentaires de la peau » une diminution de la pigmentation des taches pigmentaires localisées en particulier du nombre et/ou l'intensité des taches pigmentaires typiquement sur les zones visage, le décolleté, le cou, le dos, les épaules et/ou des mains, particulièrement des taches sur le dessus des mains. Les taches pigmentaires sont notamment les «taches brunes » ou « aged spots » lorsqu'elles sont localisées sur une partie du corps exposée aux radiations UV et/ou liées au vieillissement chrono ou UV induit, par exemple lors d'une exposition au soleil. Les taches pigmentaires incluent également les taches de rousseur, et/ou les taches post-inflammatoires et/ou qui apparaissent en réponse à des agressions, et/ou d'origine hormonales, en particulier dans le cadre d'un chloasma, et/ou d'origine médicamenteuse, et/ou pour prévenir et/ou lutter contre les manifestations pigmentaires inesthétiques accompagnant une pathologie.

L'extrait de *L. domesticum* selon l'invention est un extrait cosmétique et/ou nutraceutique ou pharmaceutique, préférentiellement dermatologique topiquement et/ou oralement acceptable. Au sens de la présente invention, on entend par « topiquement et/ou oralement acceptable », un ingrédient adapté à une application respectivement par voie topique et/ou orale, non toxique, non irritant pour la peau et n'induisant pas de réponse allergique, et qui n'est pas instable sur le plan chimique.

L'utilisation de l'extrait selon la présente invention peut être par voie orale et/ou topique. Avantageusement, elle est par voie topique. Au sens de la présente invention, on entend par « voie topique », l'application locale directe et/ou la vaporisation de l'extrait sur la surface de la peau.

L'extrait selon l'invention est un extrait de feuilles obtenu par tout procédé classique d'extraction de plantes connu de l'Homme du métier. Les feuilles peuvent être ainsi séchées et/ou broyées avant extraction. L'extraction peut être réalisée par macération, par décoction à chaud, par broyage aux ultrasons, par broyage à l'aide d'un mixeur, par extraction en conditions subcritiques ou supercritiques (dioxyde de carbone), ou encore par simple agitation, préférentiellement sous agitation magnétique. Préférentiellement, l'extraction est réalisée par macération sous agitation magnétique.

Dans un mode de réalisation de l'invention, l'extrait de *L. domesticum* est obtenu par extraction, préférentiellement par agitation magnétique, des feuilles dans un solvant ou un mélange de solvants, de préférence un solvant polaire protique, et avantageusement dans l'eau, un alcool, un glycol, un polyol, ou un mélange eau/alcool, eau/glycol ou eau/polyol (tels que l'eau en mélange avec éthanol, glycérol et/ou butylène glycol et/ou autres glycols, tel que xylitol etc.) de 100/0 à 0/100 (p/p). Plus préférentiellement, le solvant utilisé est constitué par de l'eau seulement.

De manière préférentielle, l'extrait est obtenu par extraction aqueuse. Au sens de la présente invention, on entend par « extrait obtenu par extraction aqueuse » tout extrait obtenu par extraction avec une solution aqueuse contenant plus de 60% en poids, avantageusement au moins 70% en poids, en particulier au moins 80% en poids, plus particulièrement au moins 90% en poids, de façon particulière au moins 95% en poids, d'eau par rapport au poids total de la solution aqueuse, encore plus avantageusement ne contenant pas de glycol et de façon particulière ne contenant pas d'alcool, plus particulièrement ne contenant que de l'eau. L'extraction peut être réalisée durant une période allant de 1 heure à 20 heures, préférentiellement de 2 heures à 16 heures. Avantageusement, l'extraction est réalisée durant une période de 2 heures.

L'extraction peut être réalisée à une température comprise entre 0°C à 80 °C, préférentiellement entre 0 °C et 25 °C, plus préférentiellement entre 4 °C et 20 °C. Encore plus préférentiellement, l'extraction est réalisée à température ambiante, c'est-à-dire à 20 °C. Dans un mode alternatif de réalisation, l'extraction est réalisée à une température entre 60°C et 80°C, avantageusement à une température de 80°C.

L'extrait peut par ailleurs être obtenu à partir d'une quantité de matière sèche de 1 à 20%, avantageusement de 2 à 15%, encore avantageusement de 5 à 10%, et encore préférentiellement de 10% en poids par rapport au poids total de la matière sèche et du solvant utilisé.

L'extrait peut être obtenu sous forme liquide ou de poudre. Lorsqu'il est obtenu sous forme de poudre, il est séché, et préférentiellement dans ce cas, de la maltodextrine est ajoutée en quantité de 20 à 90%, avantageusement de 40 à 80%, encore avantageusement de 75%, en poids par rapport au poids total de la maltodextrine avant séchage et de l'extrait.

Dans un mode préférentiel de réalisation de l'invention, l'extrait est obtenu sous agitation magnétique à partir d'une quantité de 10% en poids de feuilles de *L*. *domesticum* par rapport au poids total de feuilles et de solvant, dans l'eau comme unique solvant, durant une période de 2 heures à température ambiante, c'est-à-dire à une température de 20°C. L'extrait ainsi obtenu est ensuite filtré puis de la maltodextrine est ajoutée en quantité telle qu'elle représente 75 % en poids par rapport au poids total de l'extrait et de la maltodextrine, dans des conditions décrites dans l'exemple 1a).

Dans un autre mode de réalisation de l'invention, l'extrait est obtenu sous agitation magnétique d'une quantité de 15% en poids de feuilles de *L*. *domesticum* dans l'eau comme unique solvant durant une période de 2 heures à température ambiante, c'est-à-dire à une température de 20°C. L'extrait ainsi obtenu a été filtré puis atomisé en présence de maltodextrine ajoutée au milieu à une concentration finale de 75% en poids par rapport au poids total de la maltodextrine et de l'extrait, dans les conditions décrites dans l'exemple 1b). Dans un 3^{ème} mode de réalisation de l'invention, l'extrait est obtenu sous agitation magnétique d'une quantité de 10% en poids de feuilles de *L. domesticum* dans un mélange eau/éthanol (75, 25) (p/p) durant une période de 2 heures à température ambiante, c'est-à-dire à une température de 20°C. L'extrait ainsi obtenu a été filtré puis atomisé en présence de maltodextrine ajoutée au milieu à une concentration finale de 75% en poids par rapport au poids total de la maltodextrine et de l'extrait, dans des conditions décrites dans l'exemple 1c). Dans encore un autre mode de réalisation de l'invention, l'extrait est obtenu sous agitation magnétique d'une quantité de 10% en poids de feuilles de *L*. *domesticum* dans l'eau comme unique solvant, durant une période de 2 heures à une température de 80°C. L'extrait ainsi obtenu a été filtré puis atomisé en présence de maltodextrine ajoutée au milieu à une concentration finale de 75% en poids par rapport au poids total de la maltodextrine et de l'extrait, dans des conditions décrites dans l'exemple 1d).

Avantageusement, l'extrait de feuilles de *L. domesticum* selon l'invention ne contient pas de vitamine C ni l'un quelconque de ses dérivés.

L'extrait de *L. domesticum* selon l'invention peut être utilisé seul sous forme d'ingrédient actif cosmétique ou nutraceutique ou bien dans une composition cosmétique ou nutraceutique. Lorsqu'il est utilisé seul sous forme d'ingrédient actif, l'extrait selon l'invention est préférentiellement soluble et dissout dans un solvant notamment polaire, tel que l'eau, un alcool, un polyol, un glycol, ou un de leurs mélanges, en présence ou non de glycérine. Préférentiellement, l'extrait est dissout dans l'eau comme unique solvant pour réaliser un ingrédient cosmétique facilement formulable, tel que décrit dans l'exemple 6.

Un autre objet de l'invention concerne donc encore l'utilisation de l'extrait de *L*. *domesticum* dans une composition cosmétique et/ou nutraceutique comprenant en outre au moins un excipient cosmétiquement et/ou nutraceutiquement acceptable. Dans un mode de réalisation de l'invention, l'extrait est présent dans la composition cosmétique à une concentration de 1x10⁻⁴% à 10% en poids, préférentiellement entre 1x10⁻⁴% et 5% en poids, encore préférentiellement entre 1x10⁻³% et 3% en poids par rapport au poids total de la composition, de façon particulière entre 0,001% et 0,1% en poids par rapport au poids total de la composition. On entend ici par « composition cosmétique » une composition non pharmaceutique, c'est-à-dire destinée à une utilisation non thérapeutique. Avantageusement, la composition cosmétique est donc utilisée pour diminuer la pigmentation de la peau et/ou des annexes cutanées, préférentiellement des poils, et/ou pour diminuer les taches pigmentaires de la peau, notamment en augmentant l'expression du miRNA490 et/ou en diminuant la quantité de protéine tyrosinase, préférentiellement dans les mélanocytes.

La composition cosmétique peut comprendre en outre au moins un excipient cosmétiquement acceptable. Avantageusement, le ou lesdits excipients sont choisis parmi au moins un des groupes constitués par les conservateurs, les émollients, les émulsifiants, les tensioactifs, les hydratants, les épaississants, les agents de texture, les agents filmogènes, les pigments, les stabilisants, les solubilisants, les colorants, les parfums.

Avantageusement encore, le ou les excipients sont choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les gommes de xanthane, les cires naturelles et synthétiques, les huiles végétales, les triglycérides, les insaponifiables, les phytostérols, les esters végétaux, les silicones et ses dérivés, les hydrolysats de protéines, l'huile de Jojoba et ses dérivés, les esters lipo/hydrosolubles, les betaines, les aminoxides, les extraits de plantes, les esters de Saccharose, les dioxydes de Titane, les glycines, et les parabens, et encore de préférence parmi le groupe consistant en le stéareth-2, le stéareth-21, le glycol-15 stéaryle éther, le cétéaryl alcool, le phénoxyéthanol, le méthylparaben, l'éthylparaben, le propylparaben, le butylparaben, le butylène glycol, le caprylyl glycol, les tocophérols naturels, la glycérine, le dihydroxycetyl sodium phosphate, l'isopropyl hydroxycétyl éther, le glycol stéarate, le triisononanoin, l'octyl cocoate, le polyacrylamide, l'isoparaffine, le laureth-7, un carbomer, le propylène glycol, l'hexylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8, Beeswax, les glycérides d'huile de cœur de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

La composition de l'invention peut être choisie parmi une solution, aqueuse ou huileuse, une crème ou un gel aqueux ou un gel huileux, notamment un gel douche, un lait, une émulsion, une microémulsion ou une nanoémulsion, notamment huile-dans-eau ou eau-dans-huile ou multiple ou siliconée, un masque, un sérum, une lotion, un savon liquide, un pain dermatologique, une pommade, une mousse, un patch, un produit anhydre, de préférence liquide, pâteux ou solide. Préférentiellement, la composition cosmétique selon l'invention est une crème ou un sérum.

De façon avantageuse, la composition cosmétique selon l'invention est destinée à être appliquée, avantageusement par voie topique, sur tout ou partie du corps choisi parmi les mains, le cou, le décolleté, le ventre, les bras, les cuisses, les hanches, la taille et/ou le visage et/ou les annexes cutanées, et préférentiellement les mains, le cou, le décolleté et le visage, encore préférentiellement le visage.

Dans un autre mode de réalisation de l'invention, l'extrait de *L. domesticum* selon l'invention est compris dans une composition nutraceutique comprenant en outre au moins un excipient nutraceutiquement acceptable, à une concentration de 1x10⁻⁴% à 10% en poids, préférentiellement entre 1x10⁻⁴% et 5% en poids, encore préférentiellement entre 1x10⁻³% et 3% en poids par rapport au poids total de la composition, de façon particulière entre 0,001% et 0,1% en poids par rapport au poids total de la composition. On entend par « composition nutraceutique » une composition administrable par voie orale en tant que complément alimentaire, non thérapeutique et se présentant sous la forme de gélules, capsules, poudre ou gel. Il ne s'agit donc pas d'un médicament.

La composition cosmétique et/ou nutraceutique de la présente invention peut contenir un ou plusieurs autres ingrédients actifs cosmétiques et/ou nutraceutiques, conduisant à un effet complémentaire et/ou un effet de synergie avec l'extrait selon l'invention.

Ainsi, la composition selon l'invention pourra contenir un ou plusieurs autres actifs dépigmentant et/ou éclaircissant et/ou destinés à diminuer les taches pigmentaires de la peau, tels que le niacinamide ou vitamine B3, l'arbutine, l'acide azélaique, l'acide ascorbique ou ses dérivés, une combinaison des extraits de plante *Saxifraga sarmentosa, Psidium guajava* et *Carica papaya,* commercialisée par BASF Beauty Care Solutions France sous le nom de Dermawhite™ WF, une combinaison de sulfites et d'extraits de *Camellia sinensis, Scutellaria baicalensis, Cucumis sativus, Pyrus malus,* commercialisée sous le nom de Phytolight™ BG ou une combinaison d'un extrait de pois et de sucrose dilaurate commercialisée sous le nom de Actiwhite™, ou encore des dérivés de l'acide 4-hydroxyphenoxy acétique, en particulier l'acide 2-(4-hydroxyphenoxy)-propionique commercialisé sous le nom de Radianskin™ par BASF Beauty Care Solutions France.

Parmi d'autres ingrédients actifs qui pourront être combinés à l'extrait selon l'invention, on citera des actifs dits anti-âge tels que :
- un agent stimulant la synthèse de fibronectine, en particulier un extrait de maïs, un tel extrait étant notamment commercialisé par BASF Beauty Care Solutions France sous le nom Deliner™ et le palmitoyl pentapeptide commercialisé par la société SEDERMA sous la dénomination commerciale Matrixil® ;
- un agent stimulant la formation des fibres élastiques comme un extrait d'Origanum majorana commercialisé sous le nom Dermagenist™ par la Demanderesse
- un agent stimulant l'expression du perlécane et du dystoglycane dans la matrice extracellulaire et/ou dans la membrane basale épithéliale comme par exemple un extrait de Polygonum bistorta commercialisé sous le nom Perlaura™ par BASF Beauty Care Solutions France.
- un agent de protection du facteur de croissance des fibroblastes (FGF2) de la matrice extracellulaire contre sa dégradation et/ou sa dénaturation, notamment un extrait d'Hibiscus Abelmoscus tel que décrit dans la demande de brevet au nom de la BASF Beauty Care Solutions France déposée sous le numéro FR0654316 et commercialisé par BASF Beauty Care Solutions France sous le nom Linefactor ™ et/ou un agent de stimulation de croissance des fibroblastes par exemple un extrait de soja fermenté contenant des peptides, connu sous le nom de Phytokine™ commercialisé par BASF Beauty Care Solutions France et également décrit dans la demande de brevet EP1119344B1 (Laboratoires Expanscience), et préférentiellement une combinaison de ces deux extraits ;
- un agent stimulant la synthèse de laminine, en particulier un extrait de malt modifié par biotechnologie, un tel extrait étant notamment commercialisé par BASF Beauty Care Solutions France sous le nom Basaline™ ;
- un agent stimulant l'expression et/ou l'activité de la Hyaluronane synthase 2 (HAS2) tels que les extraits végétaux décrits dans la demande de brevet FR2 893 252 A1 et en particulier un extrait aqueux de Galanga (Alpinia galanga) et commercialisé par BASF Beauty Care Solutions France sous le nom Hyalufix™;
- un agent stimulant la synthèse de lysyl oxydase like (LOXL) tel qu'un extrait de Geophila cordifolia et ceux décrits dans la demande de brevet FR2855968, et en particulier un extrait d'aneth et commercialisé par BASF Beauty Care Solutions France sous le nom Lys'lastine ™;
- un agent stimulant la synthèse d'ATP intracellulaire, notamment un extrait d'algue Laminaria digitata ;
- un actif stimulateur de collagène tel que le rétinol et/ou la vitamine C ;
- un actif inhibiteur des métalloprotéinases (MMP) telles que plus particulièrement les MMP 1, 2, 3, 9 tel que les rétinoïdes et dérivés, les oligopeptides et les lipopeptides, les lipoaminoacides, l'extrait de feuilles d'Argania spinosa commercialisé par BASF Beauty Care solutions France SAS sous le nom Arganyl™; le lycopène ; les isoflavones, la quercetine, le kaempferol, l'apigénine.
- un agent de regonflement notamment les sphères de comblement d'acide hyaluronique commercialisé par BASF Beauty Care Solutions France sous le nom de Hyaluronic Filling Spheres ™.
- un agent pour augmenter l'expression de LOX pour augmenter l'architecture de l'épiderme comme par exemple un extrait de Cichorium intybus commercialisé sous le nom de LOX-AGE™ par BASF Beauty Care Solutions France.
- un agent pour augmenter la déglycation du collagène et/ou augmenter l'expression du collagène de type I tel qu'une combinaison d'un extrait de feuilles de Salvia miltiorrhiza et de niacine commercialisé par BASF Beauty Care Solutions France sous le nom CollRepair™.
- un agent stimulant la synthèse de lumican et de collagène tel qu'un tétrapeptide synthétique acetyl Gln Asp Val His commercialisé par BASF Beauty Care Solutions France sous le nom Dermican™et décrit dans la demande de brevet WO2005120554A1.
- un agent de protection et de stimulation de l'élastine, du collagène comme l'extrait de feuilles de Manilkara multinervis commercialisé par BASF Beauty Care Solutions France sous le nom Elestan™ et l'extrait de racine de Eperua falcata commercialisé par BASF Beauty Care Solutions France sous le nom Eperuline™
- des agents stimulant la prolifération des kératinocytes, préférentiellement utilisables dans la composition selon l'invention, notamment les rétinoïdes tels que le rétinol et ses esters, dont le palmitate de rétinyle, et le phloroglucinol.
- des agents stimulant la différenciation des kératinocytes, par exemple les minéraux tels que le calcium et les lignanes tels que le sécoisolaricirésinol, ainsi que l'extrait d'Achillea millefollium commercialisé sous le nom de Neurobiox™ par BASF Beauty Care Solutions France.

La composition cosmétique et/ou nutraceutique pourra aussi contenir un ou plusieurs ingrédients actifs anti-âges. Ainsi, l'extrait de *L. domesticum* selon l'invention pourra être combiné à un extrait d'aneth commercialisé sous le nom de Lys'lastine™ par BASF Beauty Care Solutions France, un extrait de *Polygonum bistorta* commercialisé sous le nom de Perlaura™, un extrait *d'Origanum majorana* commercialisé sous le nom Dermagenist™, un extrait de pulpe d'Argan commercialisé sous le nom de Argassential™ ou un extrait de *Cichorium intybus* commercialisé sous le nom de LOX-AGE™, un extrait de feuilles de *Manilkara multinervis* commercialisé par BASF Beauty Care Solutions France sous le nom Elestan™, un extrait de racine de *Eperua falcata* commercialisé par BASF Beauty Care Solutions France sous le nom Eperuline™ ou bien encore un extrait d'huile d'Argan commercialisé sous le nom de Arganyl™ par BASF Beauty Care Solutions France.

Comme agents apaisants entrant dans la composition de l'invention, on peut utiliser les triterpènes pentacycliques, l'acide ursolique et ses sels, l'acide oléanolique et ses sels, l'acide bétulinique et ses sels, les sels de l'acide salicylique et en particulier le salicylate de zinc, le bisabolol, l'allantoïne, les huiles insaturées en oméga 3, la cortisone, l'hydrocortisone, l'indométhacine et la beta méthasone, les actifs anti-inflammatoires, et notamment ceux décrits dans la demande FR2847267, en particulier l'extrait de racine de *Pueraria lobata* commercialisé sous le nom Inhipase™ par la demanderesse, les extraits de *Theobroma cacao.* Les ingrédients actifs agissant sur la microcirculation, vasoprotecteurs ou vasodilatateurs, peuvent être choisis parmi les flavonoïdes, les ruscogénines, les nicotinates, les huiles essentielles.

Un autre objet de l'invention concerne un procédé de soin cosmétique comprenant l'application, par voie topique, de l'extrait de *L. domesticum* selon l'invention ou de la composition cosmétique le comprenant, pour diminuer la pigmentation de la peau et/ou des annexes cutanés, préférentiellement des poils, et/ou diminuer les taches pigmentaires de la peau, notamment en augmentant l'expression du miRNA490 et/ou en diminuant la quantité de protéine tyrosinase, préférentiellement dans les mélanocytes.

Dans un mode de réalisation de l'invention, le procédé de soin cosmétique comprend l'application par voie topique de l'extrait selon l'invention ou de la composition cosmétique le comprenant sur tout ou partie du corps choisi parmi les mains, le cou, le décolleté, le ventre, les bras, les cuisses, les hanches, la taille et/ou le visage, et/ou les annexes cutanées, et préférentiellement les mains, le cou, le décolleté et/ou le visage, encore préférentiellement le visage.

Le procédé selon l'invention consiste ainsi en l'application, préférentiellement par voie topique, d'une composition cosmétique comprenant l'extrait de *L*. *domesticum* selon l'invention à une concentration de 1x10⁻⁴% à 10% en poids, préférentiellement entre 1x10⁻⁴% et 5% en poids, encore préférentiellement entre 1x10⁻³% et 3% en poids par rapport au poids total de la composition, de façon particulière entre 0,001% et 0,1% en poids par rapport au poids total de la composition. Avantageusement, la composition est appliquée au moins une fois par jour, préférentiellement deux fois par jour, durant une période de 30 jours, préférentiellement 56 jours.

Un autre objet encore de la présente invention concerne l'extrait de *L*. *domesticum* selon l'invention pour son utilisation pharmaceutique, préférentiellement dermatologique, préférentiellement par voie topique, dans le traitement de pathologies impliquant un dérèglement pigmentaire, telles que la maladie d'Addison, l'insuffisance hépathique, un purpura, un mélanome, une dermatosis papulosa nigra, que l'on qualifie d'hyperpigmentation pathologique. Dans un mode de réalisation de l'invention, l'extrait est présent dans une composition pharmaceutique, préférentiellement dermatologique, comprenant en outre au moins un excipient pharmaceutiquement, préférentiellement dermatologiquement, acceptable, à une concentration en poids de 1x10⁻⁴% à 10%, préférentiellement entre 1x10⁻⁴% et 5%, encore préférentiellement entre 1x10⁻³% et 3% en poids par rapport au poids total de la composition, de façon particulière entre 0,001% et 0,1% en poids par rapport au poids total de la composition.

Des exemples faisant référence à la description de l'invention sont présentés ci-après. Ces exemples sont donnés à titre d'illustration et ne sauraient limiter en aucun cas la portée de l'invention. Chacun des exemples a une portée générale. D'autre part, dans les exemples, tous les pourcentages sont donnés en poids, sauf indication contraire, la température est exprimée en degré(s) Celsius sauf indication contraire, et la pression est la pression atmosphérique, sauf indication contraire.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention.

### EXEMPLES

### Exemple 1 : Différentes méthodes de préparation de l'extrait de Lansium domesticum

Exemple 1a : Dix pour cent (10%) en poids de feuilles de *L. domesticum* ont été extraits sous agitation magnétique dans l'eau comme unique solvant durant une période de 2 heures à température ambiante, c'est-à-dire à une température de 20°C. L'extrait ainsi obtenu a été filtré puis atomisé en présence de maltodextrine ajoutée au milieu à une concentration finale de 75% en poids par rapport au poids total de la maltodextrine et de l'extrait (p/p). L'extrait ainsi obtenu est sous forme de poudre.

Exemple 1b: Quinze pour cent (15%) en poids de feuilles de *L. domesticum* ont été extrait sous agitation magnétique dans l'eau comme unique solvant durant une période de 2 heures à température ambiante, c'est-à-dire à une température de 20°C. L'extrait ainsi obtenu a été filtré puis atomisé en présence de maltodextrine ajoutée au milieu à une concentration finale de 75% en poids par rapport au poids total de la maltodextrine et de l'extrait (p/p). L'extrait ainsi obtenu est sous forme de poudre.

Exemple 1c : Dix pour cent (10%) en poids de feuilles de *L. domesticum* ont été extrait sous agitation magnétique dans un mélange eau/éthanol (75, 25) (p/p) durant une période de 2 heures à température ambiante, c'est-à-dire à une température de 20°C. L'extrait ainsi obtenu a été filtré puis atomisé en présence de maltodextrine ajoutée au milieu à une concentration finale de 75% en poids par rapport au poids total de la maltodextrine et de l'extrait (p/p). L'extrait ainsi obtenu est sous forme de poudre.

Exemple 1d : Dix pour cent (10%) en poids de feuilles de *L. domesticum* ont été extrait sous agitation magnétique dans l'eau comme unique solvant durant une période de 2 heures à une température de 80°C. L'extrait ainsi obtenu a été filtré puis atomisé en présence de maltodextrine ajoutée au milieu à une concentration finale de 75% en poids par rapport au poids total de la maltodextrine et de l'extrait (p/p). L'extrait ainsi obtenu est sous forme de poudre.

### Exemple 2 : Diminution de la quantité de protéine tyrosinase (TYR) en présence du miRNA490

*Protocole :* Des mélanocytes humains normaux, c'est-à-dire ne présentant pas de pathologie de la peau, issus d'un donneur sain normal, ont été cultivés dans un milieu spécifique M-PRO contenant K-SFM (Invitrogen) avec de la géniticine (100 µg/mL) et de la normicine (0,3%), avec ou sans ajout de miRNA490 synthétique (20 nmol/L final). Les cellules ont été cultivées à 37°C (sous 5% CO₂) puis ensemencées dans un milieu M-DIF pour l'extraction ci-dessous.

Les protéines cellulaires totales ont été extraites à partir des mélanocytes cultivés ci-dessus, avec un tampon de lyse (RIPA, Sigma) additionné d'un cocktail anti-protéases (Roche). Une quantité de 13 µg de protéines de chaque échantillon a été purifiée sur gel (NuPage Novex, Invitrogen) puis transférée sur membrane de cellulose. Les membranes ont été saturées avec un tampon Tris (5% Tris buffered Saline) durant 1 heure à température ambiante puis incubées à 4°C durant 1 nuit avec un anticorps primaire anti-TYR (sc-20035) et anti-TRP1 (sc-58438)) ou anti-actine (HRP-b- actin (A3854)) (Sigma) en tant que contrôle, suivi d'une seconde incubation durant 1 heure à température ambiante avec un anticorps secondaire. La détection a été effectuée avec le kit ECL prime (GE Amersham). La quantité de tyrosinase (TYR) a été mesurée par Western blot, les résultats sont présentés ci-après et sont la moyenne (MOY) de 3 essais (n =3).

### Résultats :

**Tableau 2**

| | MOY | ET |
|---|---|---|
| Contrôle | 100 | 1,0 |
| + miRNA490 (20 nmol/L) | 53,5 | 7,4 |

| | | |
|---|---|---|
| ET : Ecart-type ; MOY : moyenne | | |

*Conclusion :* Ces résultats ont montré que le miRNA490 a provoqué une diminution d'au moins 46,5 % de la quantité de tyrosinase détectée dans les mélanocytes humains.

### Exemple 3 : Diminution de la quantité de protéine tyrosinase (TYR) en présence de l'extrait de Lansium domesticum

### Protocole :

Des mélanocytes humains normaux, ont été cultivés dans un milieu de culture M254 (Life technologies) à une température de 37°C (5% CO₂) puis l'extrait de *L. domesticum* préparé selon l'exemple 1a) a été ajouté au milieu à une concentration finale de 8,5 x 10⁻³ % (volume/volume). Les cellules ont été lysées et la quantité de protéine totale a été mesurée par méthode BSA.

La mesure de la quantité de protéine a été mesurée par Western blot (Sally Sue, Protein Sample, San José) (n = 6).

### Résultats :

**Tableau 3**

| | MOY |
|---|---|
| Contrôle | 100 |
| Extrait de *L. domesticum* 8,5 x 10⁻³ % (v/v) | 63,11 |

| | |
|---|---|
| (n = 6 ; p<0,01 (**)) ET : Ecart-type ; MOY : moyenne | |

L'extrait de *L. domesticum* selon l'invention a permis de diminuer la quantité de tyrosinase d'au moins 37%.

### Exemple 4 : Augmentation de l'expression du miRNA490 dans des mélanocytes humains normaux en présence de l'extrait de Lansium domesticum

### Protocole :

La culture des mélanocytes a été effectuée telle que décrite dans le protocole de l'exemple 2. L'ARN total a été extrait des mélanocytes après culture (kit SV 96 total RNA Isolation System) puis transformé en ADNc par RT-PCR (kit mi SCRIPT SYBR Green PCR). L'amplification du miRNA490 (Tableau 4) a été effectuée en utilisant les amorces commerciales (Qiagen) (Références 218300 MS00004319 (Hs_miR-490_1 miScript Primer Assay) et QF00065247 (Hs_RNU1-8_QF_1 QuantiFast Probe Assay) pour le gène de référence (Numéro d'accession NR_004430.2).

**Tableau 4**

| | Séquence |
|---|---|
| miR-490-3p (MIMAT0002806 (mirBase)) | CAACCUGGAGGACUCCAUGCUG SEQ ID No 2 |

### Résultats :

Les résultats présentés dans le Tableau 5 sont la moyenne de 5 essais (n = 5)

**Tableau 5**

| | MOY | ET |
|---|---|---|
| Contrôle | 100 | 15,0 |
| Extrait de *L. domesticum* à 9 x 10⁻³ (p/p) | 143,2 | 26,8 |

| | | |
|---|---|---|
| ET : Ecart-type ; MOY : moyenne | | |

*Conclusion* : l'extrait de *L. domesticum* selon l'invention a permis d'augmenter l'expression du miRNA490 dans les mélanocytes humains d'au moins 1% et jusqu'à 85%.

### Exemple 5 : Diminution de la quantité de mélanine en présence de l'extrait de Lansium domesticum.

*Protocole* : Des mélanocytes B16 ont été cultivés dans le milieu Eagle's Minimum Essential Médium contenant 2% de sérum fœtal (Fœtal Calf Serum) durant 3 jours à 37°C sous 5% CO₂ puis l'extrait de *L. domesticum* a été ajouté à une concentration finale de 4x10⁻³ ou 9x10⁻³ en poids par rapport au poids total du milieu (p/p) et le milieu a été incubé durant 3 jours supplémentaires. Le même milieu a été incubé dans des conditions identiques sans extrait de *L. domesticum* en présence de NDP-a-MSH (Contrôle). La quantité de mélanine a été mesurée par mesure de la densité optique à 475nm.

Les résultats de la mesure de mélanine sont la moyenne de 6 essais (n=6).

### Résultats :

**Tableau 6**

| | MOY | ET |
|---|---|---|
| Contrôle | 100 | 6 |
| Extrait de *L. domesticum* 4x10⁻³ % (p/p) | 19,2 | 1,7 |
| Extrait de *L. domesticum* 9x10⁻³ % (p/p) | 21,4 | 0,6 |

| | | |
|---|---|---|
| ET : Ecart-type ; MOY : moyenne | | |

*Conclusion* : les résultats ont montré une diminution d'au moins 78% de la quantité de mélanine détectée dans les mélanocytes et jusqu'à 88,5%, en présence de l'extrait de *L. domesticum* selon l'invention.

### Exemple 6 : Ingrédient cosmétique comprenant un extrait de Lansium domesticum.

| | |
|---|---|
| Extrait de *L. domesticum* selon l'exemple 1a) | 0,1 - 10% |
| Eau | qsp 100 |

### Exemple 7 : Compositions cosmétiques comprenant l'ingrédient actif selon l'invention.

Les compositions ci-après sont préparées selon des méthodes connues de l'Homme du métier, en particulier s'agissant des différentes phases à mélanger entre elles.

*L'ingrédient cosmétique correspond à celui de l'exemple 6 ci-dessus.

**Formulation 7a :**

| | |
|---|---|
| Ingrédient cosmétique* | 0,001 - 10% |
| EDTA | 0,05 |
| Steareth-2 | 2,00 |
| Steareth-21 | 2,50 |
| Alcool cetearyl | 1,00 |
| Caprylate de propylheptyl | 15,00 |
| Hydroxyde de sodium (30 % en solution) | 0,10 |
| Mélange de phénoxyéthanol, chlorphenesin, acide benzoïque, butylène glycol, acide sorbique (Germazide ™ PBS) | 1,25 |
| Mélange de polyacrylate-X, d'isohexadécane et de polysorbate | 60 |
| (Sepigel™ SMS 60) | 4,00 |
| Eau | Qsp 100 |

**Formulation 7b : Utilisation de l'ingrédient cosmétique selon l'invention dans une formulation de type eau dans huile**

| | | |
|---|---|---|
| A | PEG30 | 3 |
| | Dipolyhydroxystéarate | |
| | Caprique Triglycérides | 3 |
| | Cetearyl Octanoate | 4 |
| | Dibutyle Adipate | 3 |
| | Huile de pépins de raisin | 1,5 |
| | Huile de jojoba | 1,5 |
| | Phenoxyéthanol, méthylparabène, propylparabène, butylparabène, éthylparabène | 0,5 |
| B | Glycérine | 3 |
| | Butylène Glycol | 3 |
| | Sulfate de magnésium | 0,5 |
| | EDTA | 0,05 |
| | Eau | qsp 100 |
| C | Cyclométhicone | 1 |
| | Diméthicone | 1 |
| D | Parfum | 0,3 |
| E | Ingrédient cosmétique* | 0,001 - 10 % |

**Formulation 7c : Utilisation des produits de l'invention dans une formulation de gels aqueux (Visage)**

| | | |
|---|---|---|
| A | Eau | qsp 100 |
| | Carbomère | 0,5 |
| | Butylène Glycol | 15 |
| | Phenoxyéthanol, méthylparabène, propylparabène, butylparabène, éthylparabène | 0,5 |
| B | Ingrédient cosmétique* | 0,001 - 10 % |

**Formulation 7d : Utilisation des produits de l'invention dans une formulation de type shampoing ou gel douche (Corps)**

| | | |
|---|---|---|
| A | Eau | qsp100 |
| B | Butylène glycol, méthylparabène, Ethylparabène, propylparabène | 0,5 |
| | Phénoxyéthanol, méthylparabène, propylparabène, butylparabène, éthylparabène | 0,5 |
| C | Acide citrique | 0,8 |
| D | Sodium Laureth Sulfate | 40,0 |
| E | Ingrédient cosmétique* | 0,001 - 10 % |

### Exemple 8: Exemple de formulation cosmétique contenant l'extrait selon l'invention destinée au soin du visage.

| | | |
|---|---|---|
| A | Glyceryl Stearate, Ceteareth-20, Ceteareth-12, Cetearyl Alcohol, Cetyl Palmitate | 6,00 |
| | Cetearyl Alcohol | 2,00 |
| | Cetearyl Isononanoate | 3,00 |
| | Isopropyl Myristate | 5,00 |
| | Propylheptyl caprylate | 2,00 |
| | Dicaprylyl Carbonate | 2,00 |
| | Diméthicone | 1,00 |
| | Tocophérol | 0,20 |
| B | Eau | 67,25% |
| | Propylène Glycol, Phénoxyéthanol, Chlorphénésin, Methylparabène | 2,50 |
| | Disodium EDTA | 0,05 |
| | Butylène Glycol | 3,00 |
| C | Gomme de xanthane | 0,2 |
| | Stearoyl glutamate de sodium | 0,5 |
| D | Eau | 5 |
| | Extrait selon l'exemple 1a) | 0,3% |

### Exemple 9 : Analyse in vivo de la diminution de la quantité de mélanine et de la surface des tâches pigmentaires de la peau en présence d'un extrait de L. domesticum.

*Protocole* : La diminution de quantité de mélanine et de la surface des tâches pigmentaires a été évaluée par technique de siascopie (Siascope®) au niveau de la peau du visage d'une population de 25 femmes âgées de 18 à 70 ans d'origine asiatique (Phototype III, IV), présentant une coloration non uniforme du visage et des tâches pigmentaires.

L'application de la formulation cosmétique de l'exemple 8, contenant 0,3% en poids de l'extrait de *L. domesticum* préparé selon l'exemple 1a) par rapport au poids total de la formulation, ou de la même formulation sans extrait dite placebo a été effectuée sur les hémivisages de la population durant une période de 56 jours. Les résultats sont présentés sur une base 100 (T0) et correspondent à un pourcentage de diminution au bout de 56 jours d'application.

### Résultat :

**Tableau 7 : Diminution de la surface des tâches pigmentaires (%)**

| | T0 | T56 | T56/T0 |
|---|---|---|---|
| Placebo | 100 | 95 | -5 |
| Extrait de *L. domesticum* Ex. 1a) 0,3% | 100 | 77 | -23** |

| | | | |
|---|---|---|---|
| (Test de Student : **p<0,01) | | | |

*Conclusion* : l'extrait de *L. domesticum* a diminué la surface des tâches pigmentaires de 23% au bout de 56 jours d'application, et de 18% en comparaison du placebo.

**Tableau 8 : Diminution de la quantité de mélanine (%)**

| | T0 | T56 | T56/T0 |
|---|---|---|---|
| Placebo | 100 | 83 | -7 |
| Extrait de *L. domesticum* Ex. 1a) 0,3% | 100 | 77 | -23** |

| | | | |
|---|---|---|---|
| (Test de Student : ** p<0,01) | | | |

*Conclusion* : l'extrait de *L. domesticum* a diminué la quantité de mélanine de 23% au bout de 56 jours d'application, et de 16% en comparaison du placebo.

## Revendications

1. Utilisation cosmétique d'un extrait de feuilles de *Lansium domesticum* pour diminuer la pigmentation de la peau et/ou des annexes cutanées et/ou diminuer les taches pigmentaires de la peau.

2. Utilisation de l'extrait selon la revendication 1 telle que l'extrait est obtenu par extraction dans l'eau, un alcool, un glycol, ou un polyol, un mélange eau/alcool, eau/glycol ou eau/polyol de 100/0 à 0/100 (p/p) .

3. Utilisation de l'extrait selon la revendication 1 ou 2 telle que l'extrait est obtenu par extraction aqueuse.

4. Utilisation de l'extrait selon l'une quelconque des revendications 1 à 3 telle que l'extrait est présent dans une composition cosmétique comprenant au moins un excipient cosmétiquement acceptable à une concentration de 1x10⁻⁴% à 10% en poids par rapport au poids total de la composition.

5. Utilisation de l'extrait selon l'une quelconque des revendications 1 à 3 telle que l'extrait est présent dans une composition cosmétique comprenant au moins un excipient cosmétiquement acceptable à une concentration de 1x10⁻⁴% à 5% en poids par rapport au poids total de la composition.

6. Utilisation de l'extrait selon l'une quelconque des revendications 1 à 3 telle que l'extrait est présent dans une composition cosmétique comprenant au moins un excipient cosmétiquement acceptable à une concentration de 1x10⁻³% à 3% en poids par rapport au poids total de la composition..

7. Utilisation de l'extrait selon l'une quelconque des revendications 1 à 6 telle que l'extrait augmente l'expression du miRNA490 et/ou diminue la quantité de protéine tyrosinase.

8. Utilisation de l'extrait selon l'une quelconque des revendications 1 à 7 telle que l'utilisation est par voie topique.

9. Utilisation de l'extrait selon l'une quelconque des revendications 1 à 3 telle que l'extrait est présent dans une composition nutraceutique comprenant au moins un excipient nutraceutiquement acceptable à une concentration de 1x10⁻⁴% à 10% en poids par rapport au poids total de la composition.

10. Utilisation de l'extrait selon l'une quelconque des revendications 1 à 3 telle que l'extrait est présent dans une composition nutraceutique comprenant au moins un excipient nutraceutiquement acceptable à une concentration de1x10⁻⁴% à 5% en poids par rapport au poids total de la composition.

11. Procédé de soin cosmétique comprenant l'application par voie topique d'un extrait de feuilles de *Lansium domesticum* ou d'une composition cosmétique le comprenant pour diminuer la pigmentation de la peau et/ou des annexes cutanés, et/ou diminuer les taches pigmentaires de la peau.

12. Procédé de soin cosmétique selon la revendication 11 comprenant l'application par voie topique de l'extrait ou de la composition cosmétique le comprenant sur tout ou partie du corps choisi parmi les mains, le cou, le décolleté, le ventre, les bras, les cuisses, les hanches, la taille et/ou le visage et/ou les annexes cutanées.

13. Extrait de feuilles de *Lansium domesticum* pour son utilisation pharmaceutique dans le traitement de pathologies impliquant un dérèglement pigmentaire préférentiellement une hyperpigmentation pathologique telles que la maladie d'Addison, l'insuffisance hépathique, un purpura, un mélanome, une dermatosis papulosa nigra.

14. Extrait pour son utilisation selon la revendication 13 tel qu'il est défini dans l'une quelconque des revendications 2 et 7 à 8.

15. Extrait pour son utilisation selon l'une quelconque des revendications 13 à 14 tel qu'il est présent dans une composition pharmaceutique comprenant en outre au moins un excipient pharmaceutiquement, à une concentration en poids de 1x10⁻⁴% à 10% par rapport au poids total de la composition .

## Patentansprüche

1. Kosmetische Verwendung eines Extrakts aus Blättern von *Lansium domesticum* zur Reduzierung der Pigmentierung der Haut und/oder von Hautanhangsgebilden und/oder zur Reduzierung von Pigmentflecken der Haut.

2. Verwendung des Extrakts gemäß Anspruch 1, wobei der Extrakt mittels Extraktion in Wasser, einem Alkohol, einem Glycol oder einem Polyol, einem Gemisch aus Wasser/Alkohol, Wasser/Glycol oder Wasser/Polyol von 100/0 bis 0/100 (w/w) erhalten wird.

3. Verwendung des Extrakts gemäß Anspruch 1 oder 2, wobei der Extrakt mittels wässriger Extraktion erhalten wird.

4. Verwendung des Extrakts gemäß einem der Ansprüche 1 bis 3, wobei der Extrakt in einer kosmetischen Zusammensetzung vorliegt, umfassend wenigstens einen kosmetisch verträglichen Träger mit einer Konzentration von 1x10⁻⁴ Gew.-% bis 10 Gew.-% bezogen auf das Gesamtgewicht der Zusammensetzung.

5. Verwendung des Extrakts gemäß einem der Ansprüche 1 bis 3, wobei der Extrakt in einer kosmetischen Zusammensetzung vorliegt, umfassend wenigstens einen kosmetisch verträglichen Träger mit einer Konzentration von 1x10⁻⁴ Gew.-% bis 5 Gew.-% bezogen auf das Gesamtgewicht der Zusammensetzung.

6. Verwendung des Extrakts gemäß einem der Ansprüche 1 bis 3, wobei der Extrakt in einer kosmetischen Zusammensetzung vorliegt, umfassend wenigstens einen kosmetisch verträglichen Träger mit einer Konzentration von 1x10⁻³ Gew.-% bis 3 Gew.-% bezogen auf das Gesamtgewicht der Zusammensetzung.

7. Verwendung des Extrakts gemäß einem der Ansprüche 1 bis 6, wobei der Extrakt die Expression der miRNA490 erhöht und/oder die Menge des Proteins Tyrosinase verringert.

8. Verwendung des Extrakts gemäß einem der Ansprüche 1 bis 7, wobei die Verwendung topisch erfolgt.

9. Verwendung des Extrakts gemäß einem der Ansprüche 1 bis 3, wobei der Extrakt in einer nutrazeutischen Zusammensetzung vorliegt, umfassend wenigstens einen nutrazeutisch verträglichen Träger mit einer Konzentration von 1x10⁻⁴ Gew.-% bis 10 Gew.-% bezogen auf das Gesamtgewicht der Zusammensetzung.

10. Verwendung des Extrakts gemäß einem der Ansprüche 1 bis 3, wobei der Extrakt in einer nutrazeutischen Zusammensetzung vorliegt, umfassend wenigstens einen nutrazeutisch verträglichen Träger mit einer Konzentration von 1x10⁻⁴ Gew.-% bis 5 Gew.-% bezogen auf das Gesamtgewicht der Zusammensetzung.

11. Verfahren zur kosmetischen Pflege, umfassend die topische Applikation eines Extrakts aus Blättern von *Lansium domesticum* oder einer den Extrakt enthaltenden kosmetischen Zusammensetzung zur Reduzierung der Pigmentierung der Haut und/oder von Hautanhangsgebilden und/oder zur Reduzierung von Pigmentflecken der Haut.

12. Verfahren zur kosmetischen Pflege gemäß Anspruch 11, umfassend die topische Applikation des Extrakts oder der den Extrakt enthaltenden kosmetischen Zusammensetzung auf den gesamten Körper oder einen Teil des Körpers, ausgewählt aus Händen, Hals, Dekolleté, Bauch, Armen, Schenkeln, Hüften, Taille und/oder Gesicht und/oder Hautanhangsgebilden.

13. Extrakt aus Blättern von *Lansium domesticum* zur pharmazeutischen Verwendung in der Behandlung von Krankheiten, die eine Pigmentstörung, vorzugsweise eine pathologische Hyperpigmentierung umfassen, wie Morbus Addison, Leberinsuffizienz, Purpura, Melanom, Dermatosis papulosa nigra.

14. Extrakt zur Verwendung gemäß Anspruch 13, wie in einem der Ansprüche 2 und 7 bis 8 definiert.

15. Extrakt zur Verwendung gemäß einem der Ansprüche 13 bis 14, wobei der Extrakt in einer pharmazeutischen Zusammensetzung vorliegt, umfassend außerdem wenigstens einen pharmazeutischen Träger mit einer Konzentration von 1x10⁻⁴ Gew.-% bis 10 Gew.-% bezogen auf das Gesamtgewicht der Zusammensetzung.

## Claims

1. Cosmetic use of a *Lansium domesticum* leaf extract for decreasing the pigmentation of the skin and/or of the skin appendages and/or decreasing the pigment spots on the skin.

2. Use of the extract according to Claim 1 such that the extract is obtained by extraction in water, an alcohol, a glycol or a polyol, or a water/alcohol, water/glycol or water/polyol mixture of 100/0 to 0/100 (w/w).

3. Use of the extract according to Claim 1 or 2 such that the extract is obtained by aqueous extraction.

4. Use of the extract according to either one of Claims 1-3 such that the extract is present in a cosmetic composition comprising at least one cosmetically acceptable excipient at a concentration of from 1×10⁻⁴% to 10% by weight relative to the total weight of the composition.

5. Use of the extract according to either one of Claims 1-3 such that the extract is present in a cosmetic composition comprising at least one cosmetically acceptable excipient at a concentration of from 1×10⁻⁴% to 5% by weight relative to the total weight of the composition.

6. Use of the extract according to either one of Claims 1-3 such that the extract is present in a cosmetic composition comprising at least one cosmetically acceptable excipient at a concentration of from 1×10⁻³% to 3% by weight relative to the total weight of the composition.

7. Use of the extract according to any one of Claims 1 to 6 such that the extract increases the expression of miRNA490 and/or decreases the amount of tyrosinase protein.

8. Use of the extract according to any one of Claims 1 to 7 such that the use is topical.

9. Use of the extract according to either one of Claims 1-3 such that the extract is present in a nutraceutical composition comprising at least one nutraceutically acceptable excipient at a concentration of from 1×10⁻⁴% to 10% by weight relative to the total weight of the composition.

10. Use of the extract according to either one of Claims 1-3 such that the extract is present in a nutraceutical composition comprising at least one nutraceutically acceptable excipient at a concentration of from 1×10⁻⁴% to 5% by weight relative to the total weight of the composition.

11. Cosmetic care process comprising the topical application of a *Lansium domesticum* leaf extract or of a cosmetic composition comprising same, for decreasing the pigmentation of the skin and/or of the skin appendages, and/or decreasing the pigment spots on the skin.

12. Cosmetic care process according to Claim 11, comprising the topical application of the extract or of the cosmetic composition comprising same on all or part of the body chosen from the hands, the neck, the neckline, the stomach, the arms, the thighs, the hips, the waist and/or the face and/or the skin appendages.

13. *Lansium domesticum* leaf extract for pharmaceutical in the treatment of pathological conditions including a pigment dysregulation, preferentially a pathological hyperpigmentation such as Addison's disease, liver failure, purpura, a melanoma or dermatosis papulosa nigra.

14. Extract for use thereof according to Claim 13, such that it is defined in any one of Claims 2 and 7 to 8.

15. Extract for use thereof according to either one of Claims 13 and 14, such that it is present in a pharmaceutical composition also comprising at least one pharmaceutically acceptable excipient, at a concentration by weight of from 1×10⁻⁴% to 10%, relative to the total weight of the composition.
